# EUROPEAN PATENT SPECIFICATION

(11) **EP 2 203 136 B1**
(45) Date of publication and mention of the grant of the patent: **07.09.2016**
(21) Application number: 08845038.2
(22) Date of filing: 31.10.2008
(51) Int. Cl.: A61F 7/00

(54) **APPARATUS FOR ADJUSTING OR STABILIZING THE BODY TEMPERATURE OF A PATIENT**
VORRICHTUNG ZUR ANPASSUNG ODER STABILISIERUNG DER KÖRPERTEMPERATUR EINES PATIENTEN
APPAREIL D'AJUSTEMENT OU DE STABILISATION DE LA TEMPÉRATURE CORPORELLE D'UN PATIENT

(30) Priority: 31.10.2007 DE 102007051957
(43) Date of publication of application: 07.07.2010
(60) Divisional application: 16159746.3
(73) Proprietor: seiratherm GmbH, 91074 Herzogenaurach (DE)
(72) Inventor: Baenkler, Marc, 91074 Herzogenaurach (DE); Roth, Matthias, 85376 Giggenhausern (DE)
(74) Representative: Arnason Stellbrink
(86) International application number: PCT/EP2008/064836
(87) International publication number: WO 2009/056640

(56) References cited:
- EP-A- 1 600 186
- WO-A-02/07793
- WO-A-2007/120812
- WO-A2-2004/098675
- FR-A1- 2 306 711

## Description

The invention relates to an apparatus for adjusting or stabilizing the body temperature of a patient. Therein, patient may be understood as a human or animal patient.

The positive effect of hypothermia, i.e. the systematical lowering of the body temperature, has been investigated in many studies. The results suggest reduced tissue damage and an improvement of the patient-outcome as a consequence of lowering the body temperature during the acute phase of a patient. The basic mechanism is the general decrease of the reaction rate of (bio-)chemical reactions due to lowered temperatures (van 't Hoff's rule). A typical mode of application may include lowering the body temperature by e.g. 5° C and sustaining the lowered temperature over several hours. E.g., induced (i.e. iatrogenic) hypothermia has already become a worldwide standard by evidence based medicine criteria for patients in post-reanimation state. Successful applications of induced hypothermia may also be expected in connection with the treatment of ischemic apoplexia as animal experiments already suggest. A positive effect can also be expected for acute care of trauma patients. To avoid trembling, medication suppressing the trembling may be administered.

Beside induced hypothermia, deliberate adjustment of the body temperature can be a successful approach of fighting hyperthermic states such as fever.

Further, there may be other advantageous applications of adjusting body temperature by heating, such as regaining normothermia for a person suffering hypothermia or being subject to cooling therapeutic measures.

The technical fluid known from the prior art in order to adjust the body temperature of a patient include cooling (or heating) blankets and cooling sleeves. With the devices known from the prior art, it is difficult or not possible at all to maintain a sufficiently stable body temperature of a patient. For example, cooling blankets and cooling sleeves are not effective if a large percentage of the body area is bandaged. Further, they may hamper other actions performed by medical personnel, for example if the position of the patient needs to be changed or if a body area covered by such a cooling device needs to be accessed.

Cooling catheters are also known from the prior art, wherein heat is transferred from central venous blood through- the wall of a central venous catheter to a heat carrier medium inside the catheter. This method is highly invasive and surface limited in its effectiveness due to limited heat transfer area of the catheter.

Prior Art document WO2004/098675 discloses a system for controlling the temperature of a fluid to be delivered to a patient.

The invention is as defined in the appended claims.

It is therefore an object of the present invention to control the body temperature and to maintain a sufficiently stable body temperature of a patient over a sufficient period of time in a safe, convenient and effective manner. This object is attained with the subject matter as recited in the claims.

Thus, the adjustment and stabilization of the body temperature of a patient is achieved by an actively controlled infusion of fluid of a preferably known and/or controlled temperature, employing a feedback control with body temperature being a measured variable and fluid flow being an actuating variable.

The present invention allows to actively and effectively control a patient's body temperature over a lang period of time without the need of medical personnel being constantly present.

According to a particularly preferred embodiment of the present invention, the adjustment and stabilization of the body temperature of a patient is achieved by an actively controlled balance of volume flows of infusion fluids provided at temperatures differing from one another. The volume flows result in a combined volume flow at a suitable temperature being continuously infused into the patient. The infusion fluids usable in connection with the present invention include regular isotonic sodium-chloride fluid as weil as almost the entire variety of fluids to be infused into emergency and intensive care patients anyway for various medical purposes, such as compensation offluid loss, providing nutrients, administering pharmaceuticals etc.

An apparatus according to the invention preferably comprises means for mounting a flexible tube, preferably a flexible tube which is branched in at least two proximal tubes and a distal tube, such as, for example, a plain Y-shaped tube made of a flexible synthetic material. The preferred apparatus comprises temperature sensors for measuring the respective temperature of the infusion fluids which are channelled through the proximal tubes to the distal tube and further into the patient. The temperature sensors are preferably adapted to be arranged an the proximal tubes but may as weil be adapted for measuring the temperature of the infusion fluids within respective fluid containers, from which the latter are to enter the proximal tubes. In addition or as an alternative to said temperature sensors a single temperature sensor adapted to be arranged on the distal tube may be provided. The apparatus further comprises a control unit comprising an input channel for reading in measurement data representing the patient's body temperature, and further comprising means for controlling, depending on said measurement data, flow proportions of the infusion fluids. The flow proportions preferably are mixing proportions, i.e. a common flow is created by merging flows of the infusion fluids provided. Alternatively, the flow proportions may be achieved by merely alternating the flows of the infusion fluids provided at controllable time intervals. The measurement data representing the patient's body temperature may be provided by any suitable means for measuring a patient's body temperature and outputting a respective signal.

Instead of a branched tube separate proximal tubes and distal tube connected to a mixing chamber may be employed.

Advantageously, the control characteristic of the control unit may be programmable. The control characteristic of the control unit may be dependent on a variety of input parameters such as the total amount of infusion fluid to be channelled into the patient per period of time (minute, hour or day), both long term and short term, or a respective range, the amount of a particular infusion fluid to be channelled into the patient per period of time (minute, hour or day), both long term and short term, or a respective range, the desired body temperature of the patient or a respective range, a minimum and/or maximum infusion temperature etc. The ambient temperature may also be included as an influencing quantity in the control scheme. For this, an ambient temperature sensor may be provided in the apparatus setup. Preferably, defined temperature/time-profiles can be preset and/or stored. Preferably, the control unit comprises display means and/or other output means to supply data such as required "cooling energy" (i.e. heat to be transferred), calculated body temperature without cooling, temperature profiles, comparison between target values and actual values and the like. Advantageously, additional features may be implemented such as WLAN, Bluetooth, USB or other communication interfaces for transferring measurement and control data, a printer for treatment documentation purposes and the like.

According to an advantageous embodiment, the control unit is adapted to calculate the required volume flows at respective infusion fluid inputs and output. Advantageously the infusion fluid flows (temperature and/or flow rate thereof) can be continuously controlled. Preferably, the control unit may further be adapted to calculate and control a rate of required temperature changes, i.e. it may be controlled how fast a desired temperature change is to be carried out.

According to one particularly advantageous embodiment the control unit can be programmable to control a more complex infusion management also controlling dosages of various substances. For this purpose, the apparatus may be equipped to control volume flows of multiple infusion fluids differing from one another in composition. In a particularly advantageous embodiment, the apparatus is adapted to merge various infusion components in desired mixing proportions and adjusts the temperature downstream from the mixing by employing heating and/or cooling components.

Advantageously, the fixing means for mounting the flexible tube include clips or hose clamps. Preferably, the fixing means comprise a crank, gate, guide plate or the like to ensure proper positioning of the tube and locking means such as a slider, a lock bar, a rotatable locking member or the like.

According to a particularly preferred embodiment the apparatus further comprises at least one cooling component. Even if an infusion fluid is already provided at a low temperature in its respective container, it may unfavorably heat up during long application periods, in particular if the ambient temperature is high. The cooling component may advantageously cool down an infusion fluid in its respective container or in its respective tube. Providing the apparatus with a heating component for an infusion fluid may improve the response rate in case of quick desired changes of the set-point temperature. Furthermore, a heating component can of course be used if the body temperature is to be increased.

Advantageously, the control unit comprises means for controlling the cooling- and/ or heating component.

According to another advantageous embodiment the apparatus further comprises at least one holding means for holding an infusion fluid container. Preferably, the holding means comprises means for thermally insulating the infusion fluid container, for example a jacket of a foam material such as foamed polystyrene, a double jacket, preferably provided with at least one reflective surface and/or evacuated, or other insulating means known per se se from the prior art.

Advantageously, the volume flows may be controlled using squeeze valves squeezing the tube for a reduction of volume flow of the respective infusion fluid or hose pumps. Squeeze valves may be equipped with various types of squeeze members such as eccentrics, tappets, pistons and actuators, e.g. electric, pneumatic or hydraulic actuators. The control unit controls the valves or hose pumps according to a predetermined or programmable control characteristic. Instead of squeeze valves, there may also be used internal valves, which are part of the flexible tube and which are operated by actuators of the apparatus using suitable couplings. According to one advantageous embodiment, the apparatus comprises an actuator for operating a single internal mixing valve integrated in a Y connector piece connecting the proximal tubes with the distal tube.

According to another advantageous embodiment, the apparatus comprises flow rate measurement means for determining at least one of the volume flows in the proximal and/or distal tubes. Preferably, the flow measurement means comprise non-contact flow rate measurement means such as an electrocaloric flowmeter. Other flowmeter techniques may also be employed, such as ultrasound Doppler-sensors, sound reflection/absorption measurements or the like. The flow rate of an infusion fluid can also be derived from a volume balance over the container in which the respective infusion fluid is provided. Therefore, according to an advantageous embodiment, the apparatus comprises means for measuring the fill status of an infusion fluid container, such as a level meter or scales.

According to another advantageous embodiment, the apparatus further comprises means for detecting inhomogeneity in the tube. Particularly, the means for detecting inhomogeneity may comprise an optical sensor. Other sensor types for detecting homogeneity may also be suitable, such as sound reflection sensors etc. Detection of inhomogeneities can significantly improve safety of the patient, as inhomogeneities in the infusion fluid such as gas bubbles or clots can be hazardous for the patient; at low temperatures some components of infusion fluids, such as peptides, tend to clotting. Preferably, the apparatus further comprises alarm means for outputting alarm signals upon detection of an inhomogeneity. The output signal may be an audible and/or a visual signal. Further, a function for stopping a respective infusion flow upon detection of an inhomogeneity therein may be implemented.

Alarm means may also be advantageously provided for raising an alarm if a measured infusion fluid temperature or the patient's body temperature falls below or exceeds a temperature threshold value. If means for measuring the fill status of an infusion fluid container are provided, an alarm means may advantageously indicate if the respective infusion fluid container is about to be empty. Other advantageous alarm functions may include the following: significant change of the necessary cooling effort to hold the target temperature (e.g. due to the shift from hyperdynamic circulation to hypodynamic circulation in connection with a sepsis), operating errors (e.g. wrong or missing tube connections or obvious input errors)

According to yet another advantageous embodiment of the apparatus, the apparatus may comprise means for detecting the kind of infusion fluid contained in a respective container, e.g. a bar code scanner or other code reading means. The control unit may advantageously comprise a database of common infusion fluids, allowable combinations thereof, allowable temperature ranges depending on mixing proportions etc. Alarm means may then inform the operating staff when a current infusion state is outside the allowable infusion schemes, and/or the control unit may alter the current infusion state.

A flexible tube according to the present invention comprises a tubular wall and at least one thermal interconnection for locally improving heat transfer between an inner surface and an outer surface of the tubular wall. The thermal interconnection functions as a position for coupling a temperature sensor of an apparatus according to the invention as described above.

A flexible tube according to a particular preferred embodiment of the present invention is branched in at least two proximal tubes and a distal tube, such that volume flows entering the proximal tubes result in a common volume flow in the distal tube. The tube comprises a tubular wall, and at least two thermal interconnections at the proximal tubes for locally improving heat transfer between an inner surface and an outer surface of the tubular wall. Additionally or alternatively, at least one thermal interconnection may be provided at the distal tube for locally improving heat transfer between an inner surface and an outer surface of the tubular wall. Again, the thermal interconnections function as positions for coupling the temperature sensors of an apparatus according to the invention as described above.

In embodiments with more than two proximal tubes, the tube may have a tree-like structure with several bifurcations.

Advantageously, the flexible tube may have a Y-shaped (or otherwise branched) homogenous flexible body. In another advantageous embodiment a distal and two proximal flexible tube bodies are connected with each other by a Y-shaped (or otherwise branched) connector piece. For more than two proximal tubes, several connector pieces or a connector piece with more than two proximal tube connections may be provided.

Inside, the tube may advantageously have a profiled structure to improve flow characteristics.

According to an advantageous embodiment the tubular wall is thermally insulated. Preferably, the thermally insulated tubular wall comprises a foam layer. Foamed tubes can be extruded, for example, according to a method as disclosed in DE 39 21 108 C1 or similar. Tubes with foamed and unfoamed layers can be manufactured, for example, by coextrusion, wherein the foaming can be performed similar as disclosed in DE 39 21 108 C1.

For locally improving heat transfer between an inner surface and an outer surface of the tubular wall the thermal interconnections may advantageously comprise metal granulate or a continuous metal body.

To further improve heat transmission between the infusion fluid and the temperature sensor, the diameter of the tube may be reduced in the range of the thermal interconnections, as a reduction of the tube diameter causes the velocity to rise, if the flow rate is kept constant e.g. by employing a hose pump. Due to an increase of velocity of the volume flows, an improvement of the temperature measurement is achievable.

According to another advantageous embodiment the thickness of the tubular wall is locally reduced to improve heat transmission therethrough.

According to another advantageous embodiment the flexible tube is arranged in an aseptic packing. The aseptic packing ensures that the sterile flexible tube remains sterile until use. Further, parts of the tube as well as the apparatus may comprise aseptic coatings e.g. silver containing coatings.

Sometimes, medical staff may be tempted, due to cost pressure, to re-sterilize a flexible tube for a second use. However, especially for long tubing and tubes with integrated valves or the like re-sterilization is likely to leave some residue contamination. Therefore, it can be advantageous if the flexible tube comprises a structure getting destroyed or visibly damaged when trying to re-sterilize the tube. Said structure may comprise structure damageable by heating and/or water vapor.

According to another advantageous embodiment the flexible tube comprises identification means such as an RFID tag. If the apparatus is equipped with a corresponding identification reading device such as an RFID reader, the apparatus may detect if a suitable tube is connected to the apparatus or not.

According to another advantageous embodiment the flexible tube comprises a static mixer such as a helix disposed in part of the tube, e.g. of the distal tube. Other mixing means, e.g. moveable propellers or the like may also be implemented.

According to another advantageous embodiment, the tube may comprise heat exchanger means, e.g. a double-walled structure for allowing to cool an infusion fluid while flowing through the respective tube.

According to a preferred embodiment the flexible tube comprises proximal connectors at the proximal ends of the tube and a distal connector at the distal end of the tube. Preferably, the distal connector is a Luer-Taper-connector enabling the use of standard cannulae for infusion into the patient.

Generally, any of the embodiments described or options mentioned herein may be particularly advantageous depending on the actual conditions of application. Further, features of one embodiment may be combined with features of another embodiment as well as features known per se from the prior art as far as technically possible and unless indicated otherwise.

The invention will now be described in more detail. The accompanying drawings, which are schematic illustrations, serve for a better understanding of the features of the present invention. Corresponding or analagous features are marked with the same reference numerals throughout the drawings where appropriate.

Therein
- Fig. 1: is a schematic view of an apparatus according to one embodiment of the invention,
- Fig. 2: shows a flexible tube mounted on a mounting plate of the apparatus of Fig. 1,
- Fig. 3: shows a detail of Fig. 2, indicated therein by a dashed circle,
- Fig. 4a: shows a half-extended position of a valve cylinder depicted in Figs. 2 and 3 for controlling volume flow,
- Fig. 4b: shows an alternative design of a squeeze valve,
- Fig. 5a: shows a cross-sectional view of the tube of Figs. 2 and 3 with a thermal interconnection at a proximal tube, wherein the sectional plane is indicated by line A-A in Fig. 3, and
- Fig. 5b: shows the cross-sectional view of an alternative embodiment, wherein the tubular wall is thermally insulated by a foam layer.
- Figures 6a-6e: are simplified schematic views, similar to Fig. 1, of apparatuses according to other embodiments of the invention.
- Figure 7: shows a self explaining overview of the general apparatus setup and control and feedback scheme applicable to the embodiment of Fig. 1 and similar preferred embodiments.

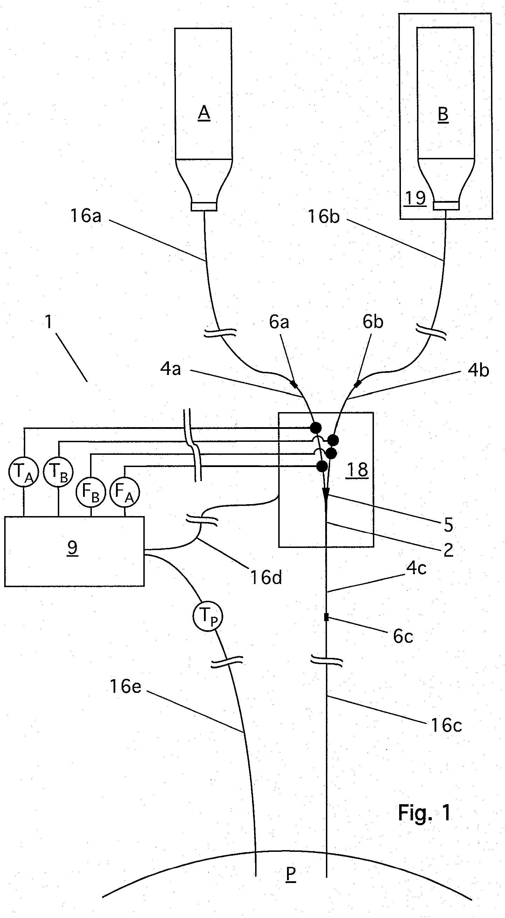

A schematic view of an apparatus according to one embodiment of the invention is depicted in Fig. 1. A flexible tube 2 is mounted on a baseplate 18. The tube 2 is branched in two proximal tubes 4a, 4b and a distal tube 4c. The flexible tube 2 comprises proximal connectors 6a, 6b at the proximal ends of the proximal tubes 4a, 4b and a distal connector 6c at the distal end of the distal tube 4c. The proximal tube 4a is connected via the proximal connector 6a and a further tube 16a to a container with an infusion fluid A. Further, the proximal tube 4b is connected via the proximal connector 6b and a further tube 16b to a container with an infusion fluid B. The infusion fluid A differs in temperature from the infusion fluid B: while infusion fluid A is kept at room temperature, infusion fluid B is cooled below ambient temperature and kept in an insulating or actively cooling housing 19. The volume flow 3a in the proximal tube 4a and the volume flow 3b in the proximal tube 4b result in a common volume flow 3c in the distal tube 4c after passing a Y-shaped connector 5 of the flexible tube 2. Further, the distal tube 4c is connected via the distal connector 6c with a further tube 16c comprising a cannula (not shown) to a patient P.

A control unit 9 is connected via interface 16d to the mounting plate 18. While an interface cable is shown for illustrative purposes, the control unit 9 and the mounting plate 18 may both be part of one apparatus housing. Further, the control unit 9 is connected via temperature line 16e to the patient P for temperature measurement. The apparatus comprises temperature measurement means TA, TB and TP. The temperature of the volume flow 3a through the proximal tube 4a is measured with a temperature measurement means TA. The temperature of the volume flow 3b through the proximal tube 4b is measured with a temperature measurement means TB. Further, the apparatus comprises flow rate controlling channels FA and FB. A flow rate of the volume flow 3a is controlled by the flow rate controlling channel FA. Likewise, a flow rate of the volume flow 3b is controlled by the flow rate controlling channel FB. The lines symbolizing the connection between the proximal tubes 4a, 4b and the temperature measurement means TA, TB and control channels FA, FB, respectively, do, in the setup depicted in Fig. 1, not constitute separate cables but will be actually wired through the interface 16d.

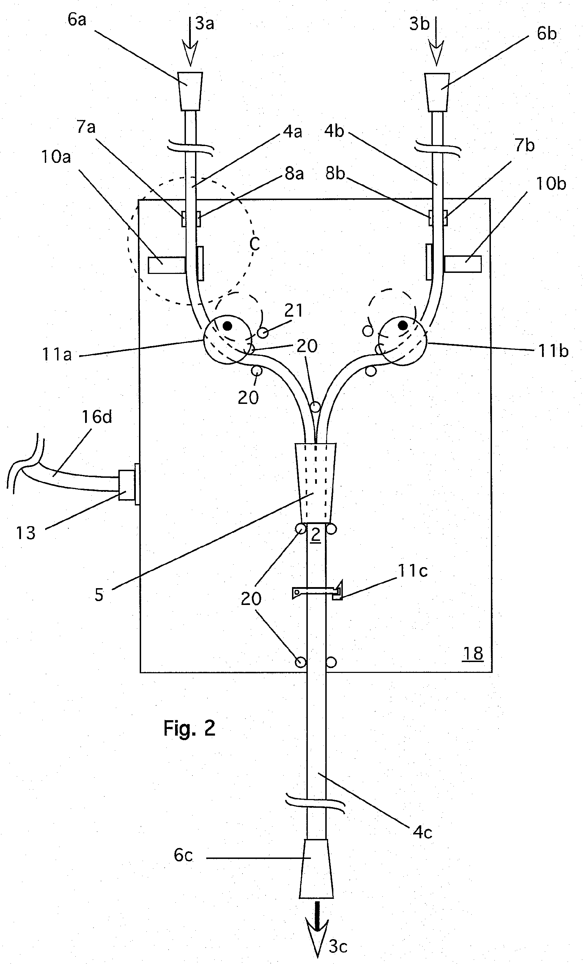

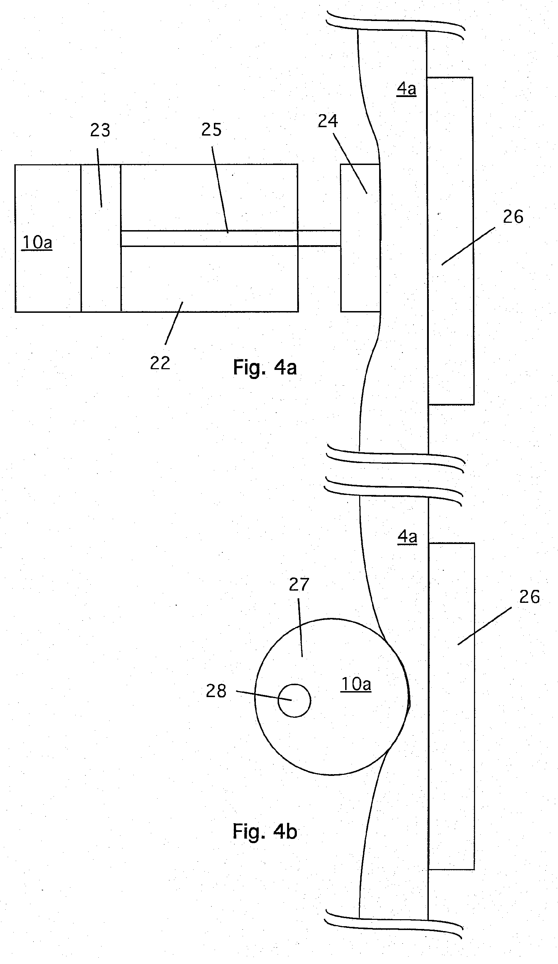

Depending on the desired body temperature of the patient the respective flow rates of the first volume flow 3a and the second volume flow 3b are controlled by the control unit 9. The control unit 9 controls valve actuators 10a, 10b for squeezing the respective proximal tubes 4a, 4b according to Fig. 4a. By squeezing the proximal tube 4a via actuator 10a (Fig. 2), the flow rate of infusion fluid A will decrease and the resulting temperature of the infusion fluid entering the patient P will decrease, as the flow proportion of the cooled infusion fluid B is thus higher. Likewise, squeezing of the proximal tube 4b via actuator 10b (Fig. 2), the flow rate of infusion fluid B will decrease and the resulting temperature of the infusion fluid entering the patient P will increase, as the flow proportion of the cooled infusion fluid B is thus lower. Both actuators 10a and 10b can be operated at the same time both in the same or in the opposite direction thus enabling, for example, a change of temperature at constant overall flow as well as a change of overall flow at a constant temperature.

Fig. 2 shows the flexible tube 2 mounted on the mounting plate 18 of Fig. 1. The flexible tube 2 is branched in two proximal tubes 4a, 4b and a distal tube 4c, such that volume flows 3a, 3b entering the proximal tubes 4a, 4b result in a common volume flow 3c in the distal tube 4c. The mounting plate 18 comprises positioning pins 20 and locking members 11 a, 11b, 11 c for proper positioning of the tube 2. The locking members 11 a, 11 b fix the proximal tubes 4a, 4b to the mounting plate 18. They are rotatable around eccentric pivot points (indicated as black dots in Fig. 2) such that they can be held in an upper position (shown by dashed lines) by respective holding pins in order to allow the tube 2 to be mounted to the mounting plate or removed therefrom and in a lower position (shown by full lines) in order to securely hold the tube 2 mounted on the mounting plate 18. The locking members 11 a, 11 b are held in the lower position by their own weight. The distal tube 4c is fixed to the mounting plate 18 by the locking member 11c, e.g. implemented as a clip. A first temperature sensor 7a is attached to the first proximal tube 4a. Opposite the first temperature sensor 7a a fixing means 8a for the first proximal tube 4a is arranged in order to establish a secure connection between the sensor 7a and the proximal tube 4b. A first valve actuator 10a for controlling volume flow 3a is further mounted next to the first proximal tube 4a. Likewise, a second temperature sensor 7b is attached to the second proximal tube 4b. Opposite to the second temperature sensor 7b a fixing means 8b for the second proximal tube 4b is arranged. A second valve actuator 10b for controlling volume flow 3b is further mounted to the second proximal tube 4b.

The flexible tube 2 comprises proximal connectors 6a, 6b at the proximal ends of the proximal tubes 4a, 4b and a distal connector 6c at the distal end of the distal tube 4c. The connectors 6a, 6b, 6c are Luer-Taper-connectors.

The mounting plate 18 further comprises a connector 13. The connector 13 links the mounting plate 18 with the control unit 9 via interface 16d. Again, the control unit 9 may as well be integrated in an apparatus housing, one side of which is the mounting plate 18.

It is to be noted that there is a wide variable of possible constructions for accommodating the tube. E.g. the tube 2 may be held in grooves instead of using positioning pins 20. An advantageous design solution may include a housing with a front or top that can be swung open to position the tube 2 underneath and then be closed. Accommodating the tube 2 in a closed housing is particularly advantageous when the infusion fluid is to be cooled or heated while flowing through the tube 2, as the cooling and/or heating components can then be disposed entirely inside the housing reducing heat loss effects.

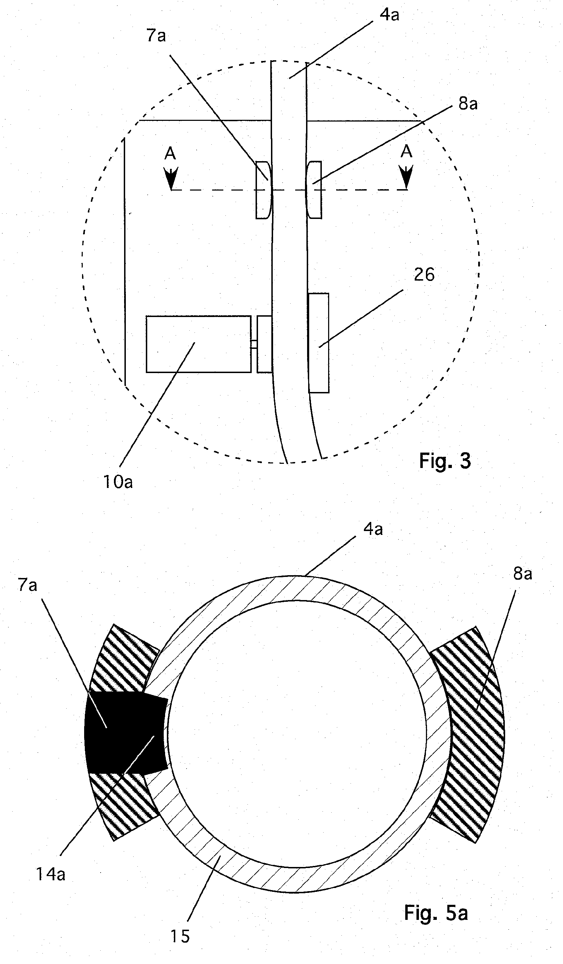

Fig. 3 shows in detail a first squeeze valve with the first valve actuator 10a and the first temperature sensor 7a of Fig. 2.

Through flow rate controlling channel FA the control unit controls the first valve actuator 10a. Likewise, a flow rate of the volume flow 3b is controlled through the flow rate controlling means FB.

Fig. 4a illustrates a half-extended position of the first valve actuator 10a shown in Fig. 3 for controlling volume flow 3a. In this example, the valve actuator 10a is implemented as a hydraulic or pneumatic cylinder 22 with an internal piston 23 connected to the squeezing piston 24 via a piston rod 24. By increasing a fluid pressure on either side of the internal piston 23, the squeezing piston can thus be moved closer to the counter plate 26 or away therefrom.

Fig. 4b shows a squeeze valve with an alternative valve actuator 10a. A disk mounted in a fixed manner to an eccentric rotatable shaft 28 forms a tappet 10 squeezing the tube 4a depending on the angular position of the shaft 28.

Fig. 5a shows a cross-sectional view A-A of the proximal tube 4a of Fig. 3 with a thermal interconnection 14a.

For locally improving heat transfer between the inner surface and the outer surface of the tubular wall 15 the thermal interconnection 14a comprises a continuous metal body locally disposed in the tubular wall 15. The thermal interconnection 14a is flush with the outer surface of the tubular wall 15. The inner surface of the tubular wall 15 comprises a thin layer overlapping the thermal interconnection 14a.

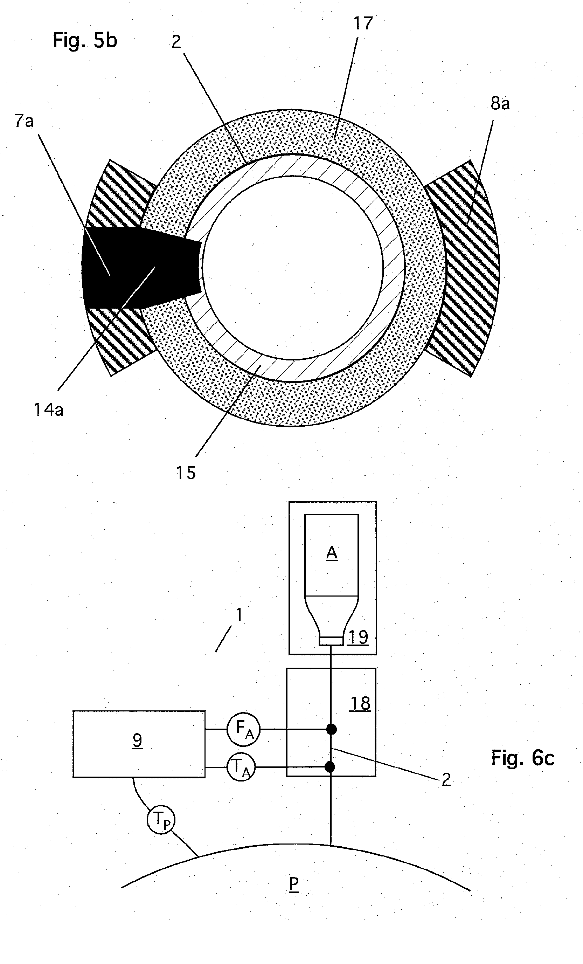

Fig. 5b shows cross-sectional view of an improved tubular wall 15 which is thermally insulated and has a thermal interconnection 14a locally disposed therein. The tubular wall 15 of the flexible tube 2 comprises a foam layer 17 for thermally insulating the tube 2.

Both in Fig. 5a and Fig. 5b a sensing element of the temperature sensor 7a is in close contact with the thermal interconnection 14a. The fixing means 8a ensures the close contact in order to improve heat transfer between the thermal interconnection and the sensing element and thus between the interior of the tube 2, 4a and the temperature sensor 7a. A thermistor may be employed as temperature sensor 7a, such as a platinum resistor, in order to yield a good sensitivity and quick response. Generally, a variety of temperature sensors known per se from the prior art can be used as temperature sensors 7a, 7b .

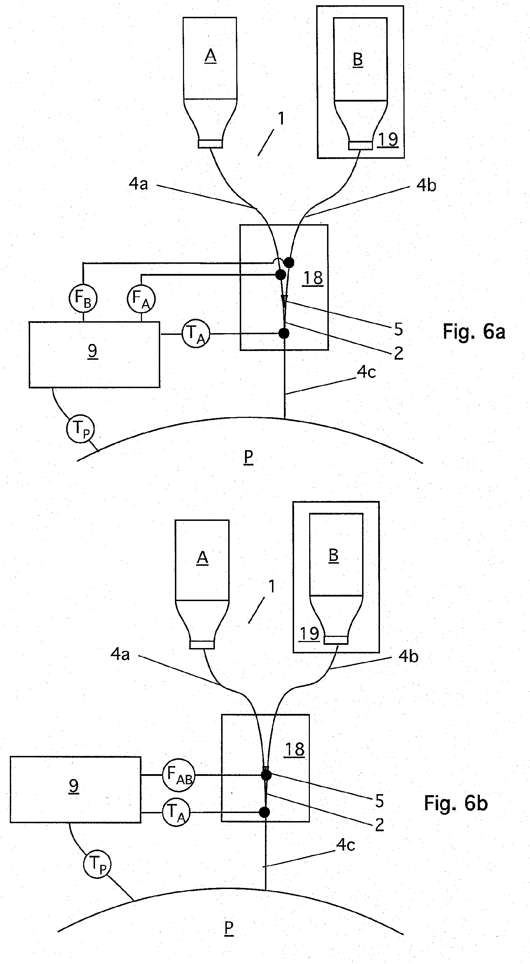

Fig. 6a is a simplified schematic view of an apparatus according to another embodiment of the invention similar Fig. 1, wherein a temperature in the distal tube 4c is measured instead of two temperatures in the proximal tubes 4a, 4b.

Fig. 6b is a simplified schematic view of an apparatus according to another embodiment of the invention similar Fig. 6a, wherein flow proportions are controlled by a single 3-way-valve integrated in a connector piece 5 connecting the two proximal tubes 4a, 4b with the distal tube 4c.

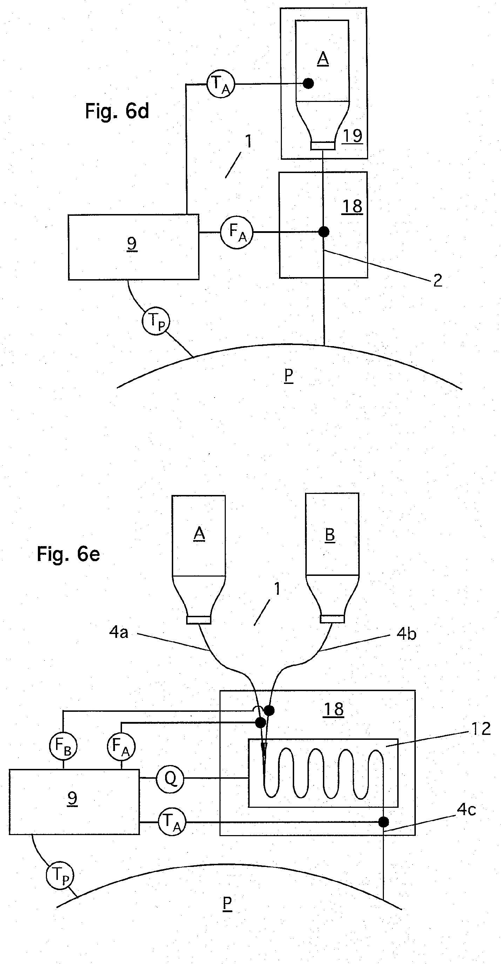

Figs. 6c and 6d show a more simple setup, wherein no mixing of infusion fluids is implemented, but merely the volume flow of the cooled single infusion fluid A is controlled by a single valve via the flow rate controlling channel FA depending on the measured body temperature TP. The tube 2 thus needs not to be branched. The temperature of the infusion fluid A can either be measured in the respective container (Fig. 6d) or in the tube (Fig. 6c).

Fig. 6e shows a different embodiment, wherein the apparatus functions as an infusion management system. The infusion fluids A and B may be provided at the same temperature but differ in composition. They are mixed at desired proportions by controlling the respective volume flows in the proximal tubes 4a, 4b via the flow rate controlling channels FA, FB, respectively. The temperature of the resulting mixture is adjusted in a heat exchanger 12 that is controlled by the control unit 9 via heat exchanger control channel Q. The heat exchanger 12 may employ a heat carrier medium such as water or silicone oil. Generally, a wide variety of cooling and/or heating techniques known per se from the prior art may be employed such as Peltier elements, an absorption chiller, a compressor chiller, electrical heater, infrared lamp etc. Depending on the cooling and/or heating technique employed, the heat exchanger control channel Q may control electrical power used for heating and/or cooling, respectively, the volume flow of a heat carrier medium etc. To improve heat transfer, the heat transfer area can be increased by increasing the length of the tube section disposed in the heat exchanger 12. For this, the respective tube section may be coiled or meandered.

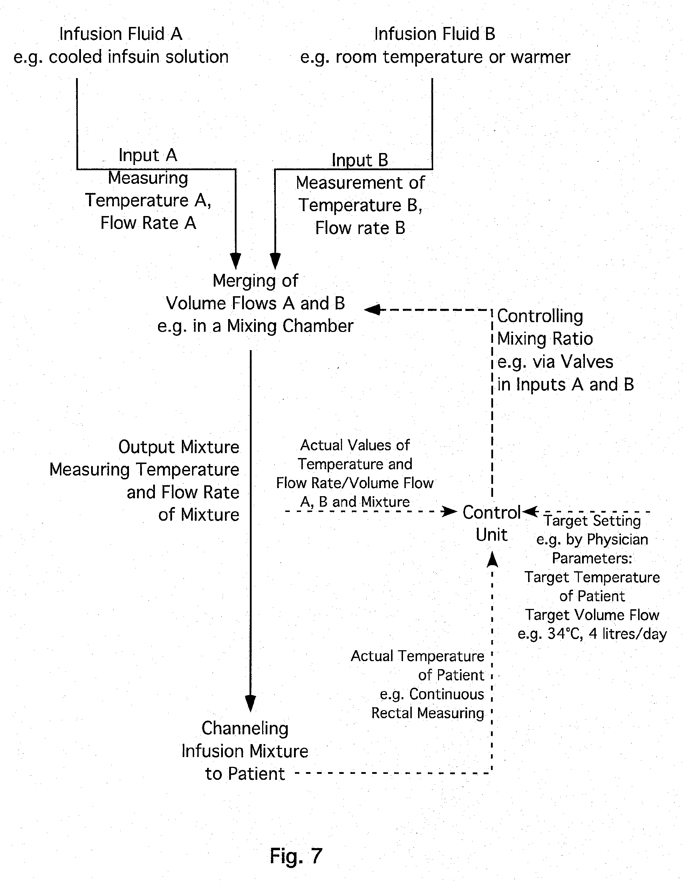

Fig. 7 shows a self explaining overview of the general apparatus setup and control and feedback scheme applicable to the embodiment of Fig. 1 and similar preferred embodiments. Target settings from the physician are input in the control unit (required temperature, daily infusion amount etc.), target settings are put in relation to actual temperature of the patient, the required mixing rate and required volume flows (flow rates) at respective infusion input and output are automatically calculated, the volume flows are controlled e.g. using valves, volume flows are continuously checked and controlled (e.g. temperature, flow rate etc.). The automatic control loop sets the patient's target temperature and keeps the body temperature at a desired level by accounting for the relevant parameters (e.g. temperature, flow rate) and actively controlling the mixing proportions.

The embodiments aspects and examples which do not fall within the scope of the claims are provided for illustrative purpose only and do not form part of the present invention.

The invention is defined in the claims as follows:

### Reference Numerals:

- A: first infusion fluid
- B: second infusion fluid
- P: patient
- 1: apparatus
- 2: flexible tube
- 3a: first proximal volume flow
- 3b: second proximal volume flow
- 3c: distal volume flow
- 4a: first proximal tube
- 4b: second proximal tube
- 4c: distal tube
- 5: V-shaped connector
- 6a: first Luer-Taper-connector
- 6b: second Luer-Taper-connector
- 6c: third Luer-Taper-connector
- 7a: first temperature sensor
- 7b: second temperature sensor
- 8a: fixing means for first proximal tube
- 8b: fixing means for second proximal tube
- 9: control unit
- 10a: first valve actuator
- 10b: second valve actuator
- 11a: first locking member
- 11b: second locking member
- 11c: third locking member
- 12: heat exchanger
- 13: connector to control unit
- 14a: first thermal interconnection
- 14b: second thermal interconnection
- 15: tubular wall
- 16a: first tube
- 16b: second tube
- 16c: tube with cannula
- 16d: interface
- 16e: temperature line to patient
- 17: insulating layer
- 18: baseplate
- 19: housing
- 20: positioning pins
- 21: holding pins
- 22: cylinder
- 23: internal piston
- 24: squeezing piston
- 25: piston rod
- 26: counter plate
- 27: tappet
- 28: shaft
- TA: temperature measurement means A
- TB: temperature measurement means B
- TP: temperature measurement means P
- FA: flow rate controlling channel A
- FB: flow rate controlling channel B
- Q: heat exchanger control channel

## Claims

1. Apparatus (1) for adjusting or stabilizing the body temperature of a patient, comprising:
at least one temperature sensor adapted to measure a temperature of infusion fluid,
a control unit (9) comprising input channels for reading in sensor readings from said temperature sensor and for reading in measurement data representing the patient's body temperature and comprising means for controlling, depending on said measurement data, a volume flow of infusion fluid,
at least two proximal tubes (4a, 4b) and a distal tube (4c) such that volume flows (3a, 3b) of at least two infusion fluids entering the proximal tubes (4a, 4b) result in a common volume flow (3c) in the distal tube (4c),
wherein said at least one temperature sensor includes a temperature sensor adapted to measure a temperature in said distal tube and/or at least two temperature sensors (7a, 7b) for measuring the temperatures of said at least two infusion fluids, and
said control unit (9) comprises means for controlling, depending on said measurement data, flow proportions of the volume flows (3a, 3b) of the infusion fluids.

2. Apparatus (1) according to claim 1, further comprising fixing means for mounting at least one flexible tube (2) for channeling infusion fluid.

3. Apparatus (1) according to claim 1 or 2, further comprising means for influencing the temperature and/or the composition of infusion fluid.

4. Apparatus (1) according to claim 3, wherein said means for influencing the temperature and/or composition of an infusion fluid comprise means for controlling the merging of at least two infusion fluids into one infusion fluid.

5. Apparatus (1) according to any of the preceding claims, wherein said apparatus (1) further comprises at least one cooling- and/ or heating component (12).

6. Apparatus (1) according to any of the preceding claims, wherein the control unit (9) comprises means for controlling the cooling- and/ or heating component (12).

7. Apparatus (1) according to any of the preceding claims, wherein said apparatus (1) further comprises at least one holding means for holding an infusion fluid container.

8. Apparatus (1) according to claim 7, wherein said holding means comprises means for thermally insulating the infusion fluid container.

9. Apparatus (1) according to any of the preceding claims, wherein said apparatus (1) comprises at least one squeeze valve (10a, 10b) and/or at least one tube pump mountable on the tube (2) for controlling the volume flow.

10. Apparatus (1) according to any of the preceding claims, wherein said apparatus (1) comprises flow rate measurement means for determining the flow rate of at least one volume flow through said tube (2).

11. Apparatus (1) according to any of the preceding claims, wherein said apparatus (1) further comprises means for detecting inhomogeneity in a volume flow through said tube (2).

12. Apparatus (1) according to any of the preceding claims comprising alarm means for outputting an alarm signal if a measured temperature of infusion fluid is outside a predetermined temperature range.

13. Apparatus (1) according to any of the preceding claims comprising a flexible tube (2), wherein said tube comprises a tubular wall (15) and at least one thermal interconnection (14a, 14b) for locally improving heat transfer between an inner surface and an outer surface of the tubular wall (15).

14. Apparatus (1) according to claim 13 wherein the flexible tube (2) comprises at least two thermal interconnections (14a, 14b) at the proximal tubes (4a, 4b) and/or at least one thermal interconnection (14a, 14b) at the distal tube for locally improving heat transfer between an inner surface and an outer surface of the tubular wall (15).

15. Apparatus (1) according to claim 13 or 14, wherein said tubular wall (15) is thermally insulated.

## Patentansprüche

1. Vorrichtung (1) zur Anpassung oder Stabilisierung der Körpertemperatur eines Patienten, umfassend:
wenigstens einen Temperatursensor, der zur Messung einer Temperatur einer Infusionsflüssigkeit ausgelegt ist,
eine Steuereinheit (9), die Eingangskanäle zum Einlesen von Sensormessungen von dem Temperatursensor und zum Einlesen von Messdaten, welche die Körpertemperatur des Patienten repräsentieren, umfasst, und die Mittel zur Steuerung, in Abhängigkeit von den Messdaten, einer Volumenströmung der Infusionsflüssigkeit umfasst,
wenigstens zwei proximale Schläuche (4a, 4b) und einen distalen Schlauch (4c), so dass die Volumenströmungen (3a, 3b) von wenigstens zwei Infusionsflüssigkeiten, die in die proximalen Schläuche (4a, 4b) eintreten, eine gemeinsame Volumenströmung (3c) im distalen Schlauch (4c) ergeben,
wobei der wenigstens eine Temperatursensor einen Temperatursensor, der zur Messung einer Temperatur in dem distalen Schlauch ausgelegt ist, und/oder wenigstens zwei Temperatursensoren (7a, 7b) zum Messen der Temperaturen von wenigstens zwei Infusionsflüssigkeiten aufweist, und
wobei die Steuereinheit (9) Mittel zur Steuerung, in Abhängigkeit von den Messdaten, der Strömungsanteile der Volumenströmungen (3a, 3b) der Infusionsflüssigkeiten umfasst.

2. Vorrichtung (1) nach Anspruch 1, ferner umfassend Fixiermittel zum Anbringen wenigstens eines flexiblen Schlauchs (2) zum Lenken von Infusionsflüssigkeit.

3. Vorrichtung (1) nach Anspruch 1 oder 2, ferner umfassend Mittel zur Beeinflussung der Temperatur und/oder der Zusammensetzung der Infusionsflüssigkeit.

4. Vorrichtung (1) nach Anspruch 3, wobei die Mittel zur Beeinflussung der Temperatur und/oder Zusammensetzung einer Infusionsflüssigkeit Mittel zur Steuerung des Zusammenfassens von wenigstens zwei Infusionsflüssigkeiten zu einer Infusionsflüssigkeit umfassen.

5. Vorrichtung (1) nach einem der vorhergehenden Ansprüche, wobei die Vorrichtung (1) ferner wenigstens eine Kühl- und/oder Heizkomponente (12) umfasst.

6. Vorrichtung (1) nach einem der vorhergehenden Ansprüche, wobei die Steuereinheit (9) Mittel zur Steuerung der Kühl- und/oder Heizkomponente (12) umfasst.

7. Vorrichtung (1) nach einem der vorhergehenden Ansprüche, wobei die Vorrichtung (1) ferner wenigstens ein Haltemittel zum Halten eines Infusionsflüssigkeitsbehälters umfasst.

8. Vorrichtung (1) nach Anspruch 7, wobei das Haltemittel Mittel zur Wärmeisolierung des Infusionsflüssigkeitsbehälters umfasst.

9. Vorrichtung (1) nach einem der vorhergehenden Ansprüche, wobei die Vorrichtung (1) wenigstens ein Quetschventil (10a, 10b) und/oder wenigstens eine Schlauchpumpe umfasst, die zur Steuerung der Volumenströmung auf dem Schlauch (2) anbringbar sind.

10. Vorrichtung (1) nach einem der vorhergehenden Ansprüche, wobei die Vorrichtung (1) Durchflussraten-Messmittel zur Bestimmung der Durchflussrate wenigstens einer Volumenströmung durch den Schlauch (2) umfasst.

11. Vorrichtung (1) nach einem der vorhergehenden Ansprüche, wobei die Vorrichtung (1) ferner Mittel zum Nachweisen von Inhomogenität in einer Volumenströmung durch den Schlauch (2) umfasst.

12. Vorrichtung (1) nach einem der vorhergehenden Ansprüche, umfassend Alarmmittel zum Ausgeben eines Alarmsignals, wenn eine gemessene Temperatur der Infusionsflüssigkeit außerhalb eines vorbestimmten Temperaturbereichs liegt.

13. Vorrichtung (1) nach einem der vorhergehenden Ansprüche, umfassend einen flexiblen Schlauch (2), wobei der Schlauch eine schlauchförmige Wand (15) und wenigstens eine thermische Verbindung (14a, 14b) zur örtlichen Verbesserung des Wärmeaustauschs zwischen einer Innenseite und einer Außenseite der schlauchförmigen Wand (15) umfasst.

14. Vorrichtung (1) nach Anspruch 13, wobei der flexible Schlauch (2) wenigstens zwei thermische Verbindungen (14a, 14b) an den proximalen Schläuchen (4a, 4b) und/oder wenigstens eine thermische Verbindung (14a, 14b) am distalen Schlauch zur örtlichen Verbesserung des Wärmeaustauschs zwischen einer Innenseite und einer Außenseite der schlauchförmigen Wand (15) umfasst.

15. Vorrichtung (1) nach Anspruch 13 oder 14, wobei die schlauchförmige Wand (15) wärmeisoliert ist.

## Revendications

1. Dispositif (1) pour ajuster ou stabiliser la température corporelle d'un patient, comprenant :
au moins un capteur de température adapté pour mesurer la température d'un fluide de perfusion,
une unité de commande (9) comprenant des canaux d'entrée pour lire des lectures de capteur provenant dudit capteur de température et pour lire des données de mesure représentant la température corporelle du patient et comprenant des moyens pour contrôler, en fonction desdites données de mesure, le débit volumique du fluide de perfusion,
au moins deux tubes proximaux (4a, 4b) et un tube distal (4c) de façon que les débits volumiques (3a, 3b) d'au moins deux fluides de perfusion entrant dans les tubes proximaux (4a, 4b) aient pour résultat un débit volumique commun (3c) dans le tube distal (4c),
dans lequel ledit au moins un capteur de température comprend un capteur de température adapté pour mesurer une température dans ledit tube distal et/ou au moins deux capteurs de température (7a, 7b) pour mesurer les températures desdits au moins deux fluides de perfusion, et
ladite unité de commande (9) comprend des moyens pour contrôler, en fonction desdites données de mesure, les proportions d'écoulement des débits volumiques (3a, 3b) des fluides de perfusion.

2. Dispositif (1) selon la revendication 1, comprenant en outre des moyens de fixation pour monter au moins un tube flexible (2) pour acheminer le fluide de perfusion.

3. Dispositif (1) selon la revendication 1 ou 2, comprenant en outre des moyens pour influencer la température et/ou la composition du fluide de perfusion.

4. Dispositif (1) selon la revendication 3, dans lequel lesdits moyens pour influencer la température et/ou la composition d'un fluide de perfusion comprennent des moyens pour contrôler le fusionnement d'au moins deux fluides de perfusion en un seul fluide de perfusion.

5. Dispositif (1) selon l'une quelconque des revendications précédentes, lequel dispositif (1) comprend en outre au moins un composant de refroidissement et/ou de chauffage (12).

6. Dispositif (1) selon l'une quelconque des revendications précédentes, dans lequel l'unité de commande (9) comprend des moyens pour contrôler le composant de refroidissement et/ou de chauffage (12).

7. Dispositif (1) selon l'une quelconque des revendications précédentes, lequel dispositif (1) comprend en outre au moins un moyen de retenue pour retenir un récipient de fluide de perfusion.

8. Dispositif (1) selon la revendication 7, dans lequel ledit moyen de retenue comprend des moyens pour isoler thermiquement le récipient de fluide de perfusion.

9. Dispositif (1) selon l'une quelconque des revendications précédentes, lequel dispositif (1) comprend au moins une vanne de serrage (10a, 10b) et/ou au moins une pompe à tube montable sur le tube (2) pour contrôler le débit volumique.

10. Dispositif (1) selon l'une quelconque des revendications précédentes, lequel dispositif (1) comprend des moyens de mesure de débit pour déterminer le débit d'au moins un écoulement volumique dans ledit tube (2).

11. Dispositif (1) selon l'une quelconque des revendications précédentes, lequel dispositif (1) comprend en outre des moyens pour détecter une inhomogénéité dans un écoulement volumique dans ledit tube (2).

12. Dispositif (1) selon l'une quelconque des revendications précédentes, comprenant des moyens d'alarme pour délivrer un signal d'alarme si une température mesurée de fluide de perfusion se trouve en-dehors d'une plage de températures prédéterminée.

13. Dispositif (1) selon l'une quelconque des revendications précédentes, comprenant un tube flexible (2), dans lequel ledit tube comprend une paroi tubulaire (15) et au moins une interconnexion thermique (14a, 14b) pour améliorer localement le transfert de chaleur entre une surface intérieure et une surface extérieure de la paroi tubulaire (15).

14. Dispositif (1) selon la revendication 13, dans lequel le tube flexible (2) comprend au moins deux interconnexions thermiques (14a, 14b) au niveau des tubes proximaux (4a, 4b) et/ou au moins une interconnexion thermique (14a, 14b) au niveau du tube distal pour améliorer localement le transfert de chaleur entre une surface intérieure et une surface extérieure de la paroi tubulaire (15).

15. Dispositif (1) selon la revendication 13 ou 14, dans lequel ladite paroi tubulaire (15) est isolée thermiquement.
